# EUROPEAN PATENT APPLICATION

(11) **EP 1 878 740 A1**
(43) Date of publication of application: **16.01.2008**
(21) Application number: 07011798.1
(22) Date of filing: 15.06.2007
(51) Int. Cl.: C07K 5/06, C07C 227/04, C07C 229/14

(54) **A process for the preparation of delapril**

(30) Priority: 13.07.2006 IT MI20061364
(71) Applicant: Dipharma Francis S.r.l., 20021 Baranzate di Bollate MI (IT)
(72) Inventor: Allegrini, Pietro, 20097 S. Donato Milanese (MI) (IT); Mantegazza, Simone, 20144 Milano (IT); Razzetti, Gabriele, 20099 Sesto S. Giovanni (MI) (IT)
(74) Representative: Minoja, Fabrizio

(57) **Abstract**

A process for the preparation of delapril and intermediates useful in its preparation.

## Description

### FIELD OF THE INVENTION

The present invention relates to a process for the preparation of delapril, namely N-[N-[(S)-1-ethoxycarbonyl-3-phenylpropyl]-L-alanyl]-N-(indan-2-yl)glycine, and intermediates useful in its preparation.

### TECHNOLOGICAL BACKGROUND

Delapril, in particular as the hydrochloride, is a medicament with anti-hypertensive action connected with its ACE-inhibiting activity.

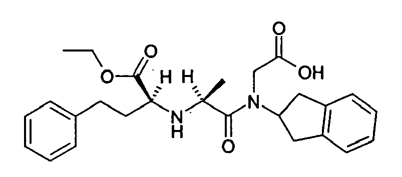

A number of synthetic methods for the preparation of delapril and its salts exist, for example as disclosed in EP 51391 and EP 281393.

As a rule, these methods make use of expensive starting products and also require the protection of the glycine carboxylic group, so that a subsequent deprotection step is necessary.

There is therefore the need for an alternative method for the preparation of delapril and its salts, which starts from easily available products, avoids unfavorable synthetic steps, particularly the protection/deprotection of functional groups, and involves less synthetic steps, under operative conditions well suited for the industrial production, thereby reducing both costs and the environmental impact.

### SUMMARY OF THE INVENTION

It has now been found a novel process for the preparation of delapril and of intermediates useful in its preparation. This process avoids the use of expensive or hardly available starting products and reactants, and requires a reduced number of steps.

### DETAILED DISCLOSURE OF THE INVENTION

The object of the invention is a process for the preparation of a compound of formula (I) (delapril) or a salt thereof, 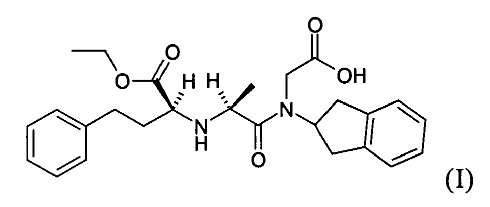 comprising the reaction of the compound of formula (II), or a salt thereof, 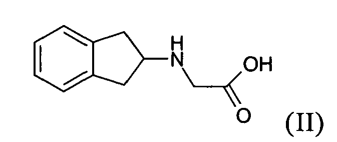 with the compound of formula (III) 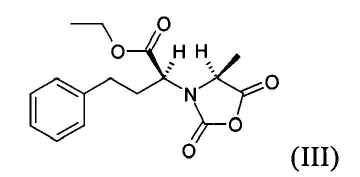 in an organic solvent, and, if desired, the conversion to a salt thereof.

The reaction between the compound (II) and the compound (III) can be carried out in the presence of a basic agent, typically an inorganic base, preferably an alkali or alkaline-earth metal hydroxide or salt, such as lithium, sodium, potassium, calcium or magnesium, e.g. sodium or potassium hydroxide, sodium or potassium carbonate; or an organic base, e.g. sodium methoxide, potassium tert-butoxide; a nitrogen organic base, e.g. a secondary or tertiary amine, particularly triethylamine, diisopropylethyl amine, 1,4-diazabiciclo-[2,2,2]-octane, or a quaternary ammonium salt, e.g. tetrabutylammonium.

Alternatively, a reaction between compound (III) and a salt of compound (II) can be carried out. A salt of compound (II) is for example an alkali or alkaline-earth metal salt, in particular lithium, sodium, potassium, calcium or magnesium, or a secondary, tertiary or quaternary ammonium salt, as obtainable with one of the basic agents reported above. Preferably a compound of formula (II) is reacted in a salified form, in particular as sodium salt.

The reaction of compound (II) or a salt thereof with compound (III) can be carried out in an organic solvent, typically an aromatic hydrocarbon, e.g. toluene or xylene; a chlorinated solvent, e.g. dichloromethane, dichloroethane, tetrachloroethylene, chlorobenzene or diclorobenzene; an ether solvent, e.g. diethyl ether, tetrahydrofuran, tert-butyl-methyl ether; an amido solvent, e.g. dimethylformamide, dimethylacetamide; a nitrile solvent, e.g. acetonitrile; a dipolar aprotic solvent such as dimethylsulfoxide, or a mixture of two or more, preferably two or three, of said solvents. The reaction is preferably carried out in tetrahydrofuran.

The stoichiometric ratio of compound (II) or a salt thereof to compound (III) can approximately range from 0.5 to 1.5, and is preferably about 1.

The reaction can be carried out at a temperature approximately ranging from -20°C to the reflux temperature of the reaction mixture, preferably approximately from 20°C to 30°C.

A compound of formula (I) can be converted to a salt thereof, typically a pharmaceutically acceptable salt as known from EP 51391, preferably the hydrochloride, according to known methods.

Compound (II) can be obtained with a process comprising the reaction of a compound (IV) or (V) 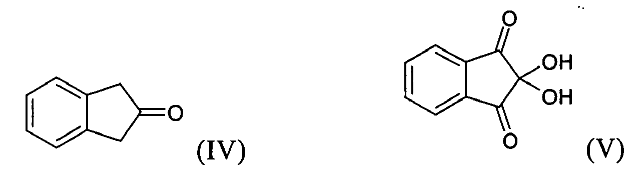 with a compound (VI) or a salt thereof, 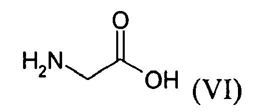 to obtain a compound of formula (VII) or (VIII) or salts thereof, respectively, 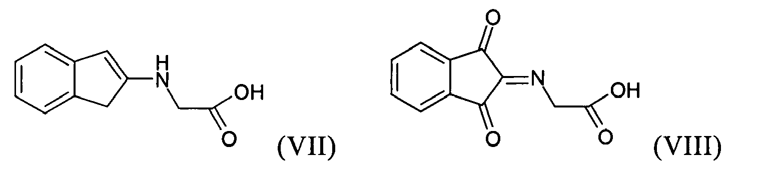 and the subsequent reduction.

A salt of compounds (II), (VI), (VII) or (VIII) is for example an alkali or alkaline-earth metal salt, in particular a lithium, sodium, potassium, calcium or magnesium salt, or a secondary, tertiary or quaternary ammonium salt, as exemplified above.

The reaction between a compound (IV) or (V) and a compound (VI) or a salt thereof can be carried out in the presence of a basic agent, e. g. an inorganic base, typically an alkali or alkaline-earth metal hydroxide, such as lithium, sodium, potassium, calcium or magnesium hydroxide, or a salt thereof, e.g. sodium or potassium carbonate; or an organic base, typically a C₁-C₆ alcoholate, e.g. sodium methoxide or potassium tert-butoxide; a nitrogen organic base, e.g. a secondary or tertiary amine, in particular triethylamine, diisopropylethylamine or 1,4-diazabicyclo-[2,2,2]-octane. Preferably the basic agent is sodium methoxide.

The molar ratio of a compound (IV) or (V) to the basic agent can range from 0.1 to 10, preferably about 0.95.

The ratio of a compound (IV) or (V) to a compound (VI) or a salt thereof can approximately range from 0.1 to 10, preferably approx from 1 to 1.1.

The reaction can be carried out in a protic solvent, e.g. a C₁-C₆ alkanol, in particular methanol, ethanol, isopropanol or butanol; in an organic ether solvent, e.g. diethyl ether or tetrahydrofuran; or in water; or in a mixture of two or more, preferably two or three, thereof; preferably in methanol.

The reaction can be carried out at a temperature approximately ranging from -20°C to the reflux temperature of the reaction mixture, preferably approximately from 20°C to 30°C.

The reduction of a compound (VII) to afford a compound (II) can be carried out with a reducing agent selected from e.g. sodium borohydride, sodium cyanoborohydride and sodium triacetoxyborohydride; or by hydrogenation in the presence of catalyst, e.g. palladium or platinum on charcoal. The reduction is preferably carried out by reaction with sodium borohydride.

The reduction of compound (VIII) or a salt thereof can be carried out by hydrogenation in the presence of catalyst, as described above.

The reduction of compounds (VII) or (VIII) can be carried out in any one of the solvents, or mixtures thereof, indicated above for the reaction between compound (IV) and compound (VI), preferably in a water / methanol mixture.

The stoichiometric ratio of a compound (VII) to the reducing agent can approximately range from 0.25 to 10, preferably about 0.28.

The reaction can be carried out at a temperature approximately ranging from 0°C to the reflux temperature of the reaction mixture, preferably approximately from 20°C to 30°C.

The preparation of compound (II) by a process comprising the reaction of a compound (V) with a compound (VI) to obtain a compound (VIII) and the subsequent reduction is a novel process and is a further object of the invention.

If desired, the resulting compound (II) can be reacted with a compound (III), according to the process reported above, to obtain delapril or a salt thereof.

Compound (III) is known and can be obtained with known methods, for example by reacting N-(1-S-carbethoxy-3-phenylpropyl)-S-alanine with carbonyl-diimidazole.

The following examples illustrate the invention.

### Example 1: Sodium (indan-2-yl)glycinate (II)

A round-bottom flask under nitrogen atmosphere is loaded with: glycine (280 g; 0.0371 mol), indanone (4.9 g; 0.0371 mol) in methanol (30 ml) and sodium methoxide (6.30 g, 0.0352 mol) in a 30% w/w methanol solution. The mixture is reacted for approximately 30 minutes at room temperature, then a solution of sodium borohydride (0.395 g, 0.0104 mol) in water (2.5 ml) at pH>12 is added. The mixture is reacted for approximately 30 minutes, then diluted with water (50 ml) and acidified to pH<2 with 37% hydrochloric acid; the aqueous phase is extracted three times with methylene chloride (150 ml). pH is adjusted above 12 with a 50% w/w aqueous solution of sodium hydroxide and the aqueous phase is concentrated under reduced pressure to obtain 10.3 g of product with 70% purity; 92% yield.

¹HNMR (300 MHz, DMSO-d₆): δ (ppm) 7.15-7.00 (m, 4H), 3.40 (m, 1H,), 2.95 (dd, 2H), 2.85 (s, 2H), 2.60 (dd, 2H).

Analogously, the title compound can be obtained by reacting compound (V) with compound (VI) according to the above procedure, and the subsequent reduction of the compound of formula (VIII), thus obtained, by catalytic hydrogenation with Pt/C.

### Example 2: Delapril hydrochloride (I)

A round-bottom flask under nitrogen stream is loaded with: N-(1-S-carbethoxy-3-phenylpropyl)-S-alanine (4.60 g; 0.0163 mol), carbonyl-diimidazole (2.90 g; 0.0179 mol) and toluene (40 ml) and the mixture is reacted for 1 hour at room temperature under stirring. The mixture is then is added with sodium (indan-2-yl)glycinate (4.90 g, 0.0163 mol; assay: 70%) and reacted for approximately 3 hours at room temperature. The solvent is evaporated off under reduced pressure, the residue is diluted with water (20 ml), acidified to pH 2-3 with 37% hydrochloric acid, diluted with methanol (50 ml) and heated to 50°C, then left to cool at room temperature. After 10-15 hours, a precipitate forms which is filtered, washed with a water/methanol mixture (5 ml, cooled to 0°C), and dried in a static dryer under vacuum at 50°C for 6-8 hours. 5.7 g of the title product are obtained; 72% yield.

¹HNMR (300 MHz, DMSO-d₆): δ (ppm) 7.36-7.17 (m, 5H), 6.96 (d, 2H), 6.74 (d, 2H), 5.22 (dd, 1H), 3.05 (m, 2 H), 2.65 (m, 2H), 2.21 (s, 3H), 2.15-1.85 (m, 2H), 1.0 (m, 12H).

### Example 3: Delapril hydrochloride (I)

A round-bottom flask under nitrogen stream is loaded with: N-(1-S-carbethoxy-3-phenylpropyl)-S-alanine (4.60 g; 0.0163 mol), carbonyl-diimidazole (2.90 g; 0.0179 mol) and tetrahydrofuran (40 ml) and the mixture is reacted for 1 hour at room temperature under stirring. The solution is added with sodium (indan-2-yl)glycinate (4.90 g, 0.0163 mol; assay: 70%) and the mixture is reacted for approximately 3 hours at room temperature, then diluted with water (20 ml) and acidified to pH 2-3 with 37% hydrochloric acid. Tetrahydrofuran is evaporated off under reduced pressure, the residue is diluted with methanol (50 ml) and the reaction mixture is heated to 50°C, then left to cool at room temperature. After 10-15 hours, a precipitate forms which is filtered, washed with a water/methanol mixture (5 ml, cooled to 0°C), then dried in a static dryer under vacuum at 50°C for 6-8 hours. 7.2 g of the title product are obtained; 90% yield.

## Claims

1. A process for the preparation of a compound of formula (I) or a salt thereof, 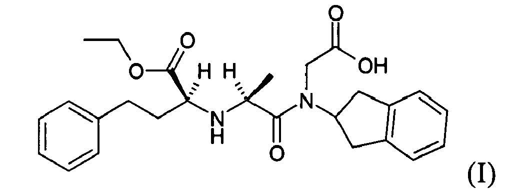 comprising the reaction of a compound of formula (II) or a salt thereof, 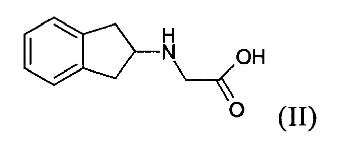 with a compound of formula (III) 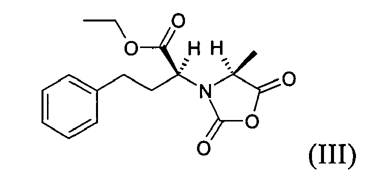 in an organic solvent and, if desired, the conversion to a salt thereof.

2. The process as claimed in claim 1, wherein the reaction is carried out in the presence of a basic agent.

3. The process as claimed in claim 1, wherein the compound of formula (II) is in the salified form.

4. The process as claimed in claim 3, wherein the compound of formula (II) is a lithium, sodium, potassium, calcium or magnesium salt, or a secondary, tertiary or quaternary ammonium salt.

5. The process as claimed in claim 1, wherein the organic solvent is selected from an aromatic hydrocarbon; a chlorinated solvent; an ether solvent; an amido solvent; a nitrile solvent; a aprotic solvent or a mixture of said solvents.

6. The process as claimed in claim 5, wherein the solvent is tetrahydrofuran.

7. The process as claimed in claim 1, wherein the stoichiometric ratio of a compound of formula (II), or a salt thereof, to a compound of formula (III) approximately ranges from 0.5 to 1.5.

8. The process as claimed in claim 7, wherein the stoichiometric ratio is approximately 1.

9. The process for the preparation of a compound of formula (II) or a salt thereof, as defined in claim 1, comprising the reaction of a compound of formula (V) 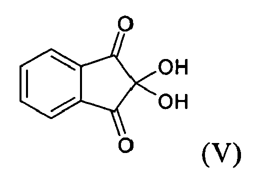 with a compound of formula (VI) or a salt thereof, 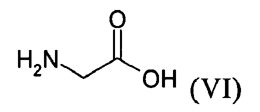 to obtain a compound of formula (VIII) or a salt thereof, 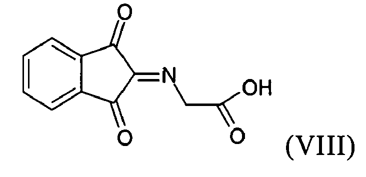 and its reduction.

10. The process as claimed in claim 9, further comprising the reaction of a compound of formula (II) or a salt thereof, with a compound of formula (III) to obtain a compound of formula (I) or a salt thereof.
